# EUROPÄISCHE PATENTSCHRIFT

(11) **EP 4 051 217 B1**
(45) Veröffentlichungstag und Bekanntmachung des Hinweises auf die Patenterteilung: **10.01.2024**
(21) Anmeldenummer: 20811976.8
(22) Anmeldetag: 02.11.2020
(51) Int. Cl.: A61K 8/04, A61K 8/9783, A61Q 19/00

(54) **KOSMETISCHE ZUSAMMENSETZUNG ENTHALTEND EINEN EXTRAKT AUS OPUNTIA FICUS INDICA**
COSMETIC COMPOSITION COMPRISING AN OPUNTIA FICUS INDICA EXTRACT
COMPOSITION COSMÉTIQUE CONTENANT UN EXTRAIT D'OPUNTIA FICUS INDICA

(30) Priorität: 01.11.2019 EP 19206753
(43) Veröffentlichungstag der Anmeldung: 07.09.2022
(73) Patentinhaber: Weleda AG, 4144 Arlesheim (CH)
(72) Erfinder: DA SILVA, Alissa Karyne, 4147 Aesch (BL) (CH); HÄNNI-CIUNEL, Katarzyna, 4312 Magden (CH); HEIZLER, Daniel, 4054 Basel (CH); IDOUX, Alicia, 4112 Bättwil (CH); KAPFER, Hélène Thérèse Anne, 68300 Saint Louis (FR); SCHMITT, Meike, 79312 Emmendingen (DE)
(74) Vertreter: Simandi, Claus
(86) Internationale Anmeldenummer: PCT/EP2020/080712
(87) Internationale Veröffentlichungsnummer: WO 2021/084136

(56) Entgegenhaltungen:
- WO-A1-2010/081839
- KR-A- 20030 023 398
- KR-A- 20080 029 035
- KR-A- 20130 032 420
- Anonymous: "HPC2_2017", , 1. März 2017 (2017-03-01), Seite 25, XP055686377, Gefunden im Internet: URL:http://www.teknoscienze.com/Contents/R iviste/Sfogliatore/HPC2_2017/27/ [gefunden am 2020-04-16]
- GABRIEL AZEVEDO DE BRITO DAMASCENO ET AL: "Use of Opuntia ficus-indica (L.) Mill extracts from Brazilian Caatinga as an alternative of natural moisturizer in cosmetic formulations", BRAZILIAN JOURNAL OF PHARMACEUTICAL SCIENCES, Bd. 52, Nr. 3, 1. September 2016 (2016-09-01), Seiten 459-470, XP055685830, DOI: 10.1590/s1984-82502016000300012

## Beschreibung

Die Erfindung betrifft eine kosmetische Zusammensetzung enthaltend einen hydrokolloidalen Extrakt aus Opuntia ficus indica, wobei gezielt Schleimstoffe angereichert sind. Weiterhin betrifft die Erfindung einen hydrokolloidalen Extrakt aus Opuntia ficus indica, ein Verfahren zur Herstellung und dessen Verwendung.

Der Feigenkaktus (Opuntia ficus indica) ist eine Pflanzenart aus der Gattung der Opuntien (Opuntia) und gehört zur Familie der Kakteengewächse (Cactaceae). Ursprünglich stammen Opuntien, auch Feigenopuntien genannt, vom amerikanischen Kontinent, wo sowohl winterharte als auch nicht winterharte Arten zwischen Kanada und Südargentinien vorkommen und mittlerweile auch in Europa heimisch sind. Der Feigenkaktus ist nicht mit der Kaktusfeige zu verwechseln, letzterer betrifft die Frucht. Opuntia ficus indica findet vielseitige Anwendungen, wie Obstanbau, Futtermittel, Gemüse, etc.

Weleda AG forscht mit modernen und in Verbindung mit anthroposophischen Methoden an vorteilhaften natürlichen Pflanzenstoffen für die Kosmetik.

Die Haut übt als das größte Organ des Menschen zahlreiche lebenswichtige Funktionen aus. Sie schützt z.B. vor Kälte, Hitze, Strahlung, dem Einwirken chemischer Substanzen und Krankheitserregern. Sofern die Haut diese Barrierefunktion nicht mehr ausreichend erfüllt, können lokale Irritationen oder auch ganzkörperliche Beschwerden erfolgen.

Die Haut, insbesondere die Epidermis, ist als Barriereorgan des menschlichen Organismus in besonderem Maße äußeren Einwirkungen unterworfen. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedenen polaren Lipiden werden im Keratinisierungsprozess freigesetzt.

In einem optimalen Zustand der Haut liegt ein ausgewogenes Verhältnis von Hautlipiden und Hautfeuchtigkeit vor. Durch dieses Gleichgewicht werden wichtige Eigenschaften der Haut, wie das Penetrationsvermögen, Wasserbindungsvermögen, Elastizität, Regenerationsfähigkeit oder die Widerstandsfähigkeit gegen Umwelteinflüsse und Noxen unterschiedlichster Art, bestimmt. Gerade die Erhaltung der Hautfeuchtigkeit ist eine kosmetische Aufgabe zur Vorbeugung der Alterung, insbesondere Faltenbildung.

Im Stand der Technik offenbart DE 20 2016 008 641 U1 die potenzielle Eignung von Opuntia ficus indica zur Verwendung als topische Hautpflegezusammensetzung.

WO 2009/058613 offenbart eine topische Zusammensetzung, enthaltend einen Opuntia ficus indica-Extrakt (Ansprüche 5, 8 und 15), welcher die Hyaluronsäure-Produktion anregt und als Feuchtigkeitsmittel (Moisturizer) verwendet werden kann. Ferner wird beschrieben, dass der Opuntia ficus indica-Extrakt auf verschiedene Weise erhalten werden kann.

KR 20030023398 A offenbart eine topische Zusammensetzung, der einen Opuntia ficus indica-Extrakt aufweist, wobei jedoch kein hydrokolloidaler Extrakt erhalten und offenbart wird, da nach einem ersten wässrigen Auszug, jedoch vor einer Mazeration mit anschließender Filtration eine überschüssige Menge an Ethanol hinzugefügt wird (dort Beispiel 1).

Weiterhin ist die kosmetische Eignung von Kaktusfeigenkernöl in EP 3 102 181 B1 beschrieben.

Die besondere Rolle der natürlichen Schleimstoffe von Opuntia ficus indica in der Kosmetik, welche vorzugsweise aus Pflanzenteilen bzw. Pflanzenzellen, jedoch vorzugsweise aus den Kladodien gewonnen werden können, werden jedoch im Stand der Technik nicht erkannt. Insbesondere wird der vorteilhafte Effekt der Schleimstoffe zur Hydration bzw. Wasserbindung unter Ausbildung eines hydrokolloidalen Extrakts im Stand der Technik nicht erkannt.

Opuntia ficus indica Extrakte sind in flüssiger Form oder als Trockenextrakte (pulverisiert) bekannt. Diese werden jedoch einfachhalber aus Presssaft von frischen Kladodien hergestellt. Durch pH Regulierung oder Zugabe von Konservierungsstoffen oder durch Überführung in eine Pulverform (mit oder ohne Träger) wird dieser Kladodiensaft mikrobiologisch stabilisiert. Dieser Presssaft enthält jedoch nur geringe Mengen an Schleimstoffen und weist daher im fluiden Zustand eine geringe Viskosität auf. Es wird daher kein hydrokolloidaler Extrakt erhalten. Die physikalischen Eigenschaften und Wirkungsprofile zweier kommerzieller Benchmarks, welche beide aus Presssaft hergestellt wurden, werden mit dem erfindungsgemäßen Gegenstand in den Beispielen verglichen.

Erfindungsgemäß sind Schleimstoffe aus Opuntia ficus indica, solche die mit höchstem Anteil aus unterschiedlich aufgebauten hochmolekularen Polysacchariden bestehen, und anteilig Polyphenole, Phenole und andere sekundäre Pflanzeninhaltsstoffe enthalten können. Ihre komplexen, hochmolekularen Strukturen sind erfindungsgemäß besonders geeignet, hohe Mengen an Wasser aufzunehmen, so dass schleimartige Kolloide und Gele bzw. Hydrokolloide ausgebildet werden können. Darüber hinaus wirken diese Schleimstoffe auf den Organismus vor allem reizmildernd, schleimhautschützend und entzündungshemmend.

Der vorliegenden Erfindung liegt daher die Aufgabe zugrunde, eine kosmetische Zusammensetzung zur topischen Verwendung aus Schleimstoffen von Opuntia ficus indica bereitzustellen als auch ein Verfahren zur Herstellung eines hydrokolloidalen Extrakts aus Opuntia ficus indica, wobei die Schleimstoffe aus Opuntia ficus indica angereichert werden können.

Überraschender Weise konnten die Erfinder feststellen, dass der reine hydrokolloidale Extrakt mit angereicherten Anteilen an Schleimstoffen eine besonders vorteilhafte kosmetische Wirkung entfaltet und auf natürliche Weise intensiv und nachhaltig zur Hautbefeuchtung beiträgt. Aufgrund der gezielten Anreicherung von Schleimstoffen kann vorteilhaft eine hohe Hydration unter Ausbildung eines hydrokolloidalen Extrakts erreicht werden.

Die Aufgabe wird daher durch ein Verfahren zur Herstellung eines hydrokolloidalen Extraktes aus Pflanzenmaterial von Opuntia ficus indica gelöst, wobei
a.) aus Pflanzenmaterial von Opuntia ficus indica ein wässriger oder vorzugsweise wässrig-alkoholischer Auszug mit maximal 40 % (m/m) Alkohol hergestellt wird, wobei der Alkohol ein C1-C4 oder C1-C3 Alkohol sein kann, jedoch vorzugsweise Ethanol (C2) ist,
b.) der erhaltene Auszug in der Wärme bei 60 - 90 Grad Celsius für eine Stunde oder länger behandelt wird,
c.) nach Abkühlung mazeriert und gefiltert wird.

In einer bevorzugten Ausführungsform kann die Mazeration mindestens über 7 Stunden als auch über Tage erfolgen, bevor die Filtration erfolgt. Weiterhin kann nach Filtration eine erneute Mazeration erfolgen. Die Mazeration kann ebenfalls als Bewegungsmazeration erfolgen, wobei die Bewegung vorzugsweise mit Hilfe von Rühren bewirkt wird.

Die Filtration gemäß Schritt c.) erfolgt vorzugsweise mit einem oder mehrere Sieben, wobei die Sieböffnungen vorzugsweise 60-300 µm betragen. Dies erlaubt das Zurückhalten von Feststoffen, so dass ein reiner und stabiler hydrokolloidaler Extrakt aus Opuntia ficus indica erhalten werden kann.

Schritt c.) kann einmal oder mehrmals wiederholt werden, ggfs. so lange bis der hydrokolloidale Extrakt vollständig ausgebildet ist, so dass sämtliche Schleimstoffe in den Extrakt erschöpfend überführt sind.

In einer besonders bevorzugten Ausführungsform beträgt der Anteil an Alkohol, insbesondere Ethanol max. 35 % (m/m) (35:65) im alkoholischen-wässrigen, insbesondere ethanolischwässrigen Auszug, vorzugsweise 30 % (30:70) oder gar 20 % (20 : 80) und weniger, jedoch vorzugsweise mehr als 10 %.

Weiterhin können C1-C4 oder C1-C3 Alkohole eingesetzt werden wie Methanol (C1), Propanol (C3), Butanol (C4), vorzugsweise jedoch Ethanol (C2). Weiterhin ist bevorzugt, dass das bevorzugt eingesetzte Ethanol 94.00 % m/m Ethanol enthält.

Ebenfalls können alkoholisch-wässrige Auszüge mit mehrwertigen Alkoholen erfolgen, insbesondere zweiwertige und dreiwertige Alkohole, insbesondere solche wie Glykol, 1,2-Propandiol, Glycerin oder Polyol.

Die Erfinder konnten feststellen, dass bereits ab 30 % Alkohol, insbesondere Ethanol die Schleimstoffe nachteilig ausfallen und nicht oder nur teilweise in den hydrokolloidalen Extrakt überführt werden können. Weiterhin ist eine rein wässrige Extraktion zwar möglich, jedoch ist die vorgenannte alkoholisch-wässrige Extraktion erfindungsgemäß bevorzugt, so dass die Schleimstoffe in den hydrokolloidalen Extrakt zeitlich effizient vollständig überführt werden können und insbesondere solche Anteile an Schleimstoffen überführt werden können, welche sekundäre Pflanzeninhaltsstoffe (Polyphenole, Phenole, Flavonoide u.a.) auch in Verbindung mit Polysacchariden aufweisen. Zudem ist nachteilig, dass der wässrige Auszug nicht im Nachhinein z.B. mit Alkohol zur Konservierung belastet werden kann, da die Schleimstoffe ausfallen.

Bei der Mazeration gemäß Schritt c.) wird das Pflanzenmaterial eine bestimmte Zeit in eine Flüssigkeit, insbesondere in der bestehenden Flüssigkeit, jedoch vorzugsweise in eine wässrigethanolische Flüssigkeit (supra) eingelegt, wobei das Mazerat (Flüssigkeit) den hydrokolloidalen Extrakt umfasst.

In einer bevorzugten Ausführungsform wird im Schritt a.) das Pflanzenmaterial getrocknet und zerkleinert. Blüten sind vorzugsweise nicht als Pflanzenmaterial zu berücksichtigen und ggfs. auszunehmen. Besonders bevorzugt ist jedoch die Verwendung von Kladodien aus Opuntia ficus indica, insbesondere getrocknete Kladodien.

In einer weiteren bevorzugten Ausführungsform kann die Wärmebehandlung gemäß Schritt b) unter Rühren erfolgen.

Weiterhin ist bevorzugt, dass die Wärmebehandlung gemäß Schritt b) bei 65 - 85 Grad Celsius, insbesondere bei 70 bis 80 Grad Celsius, insbesondere 80 Grad Celsius erfolgt.

Weiterhin ist bevorzugt, dass die Wärmebehandlung gemäß Schritt b) innerhalb 1 h erfolgt.

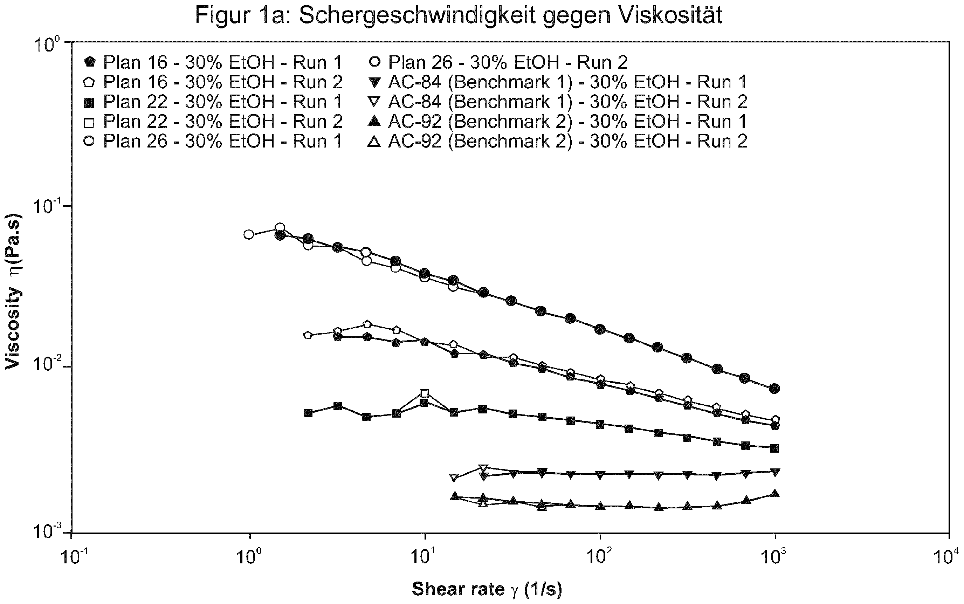

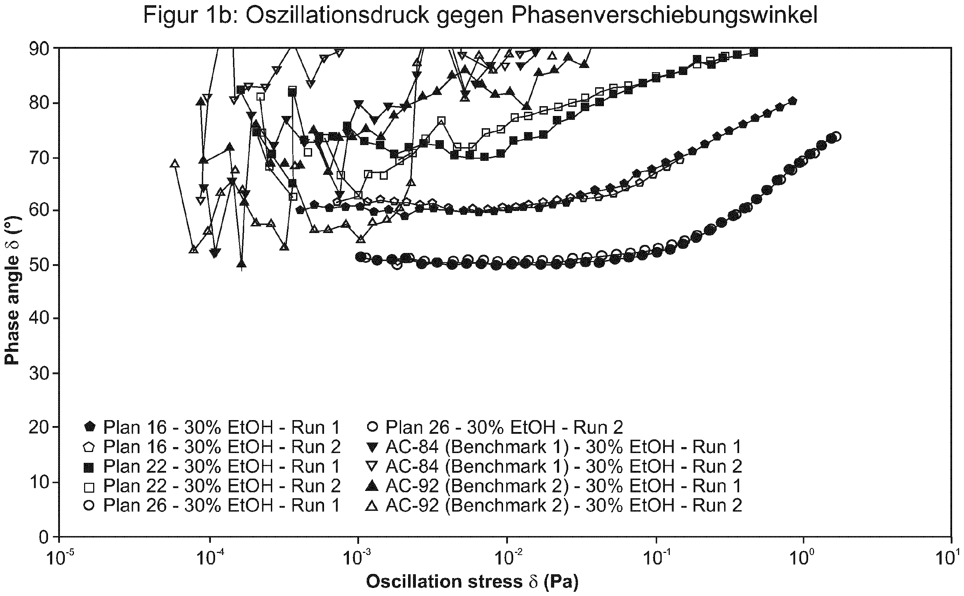

Der erfindungsgemäße hydrokolloidale Extrakt mit angereichertem Anteil an Schleimstoffen zeigt ein vorteilhaftes nicht-newtonsches Fliessverhalten in Abhängigkeit der Schergeschwindigkeit zur Viskosität als auch Oszillationsmessungen, siehe Beispiele und Figur 1a und 1b.

Dieses rheologische Verhalten ist eindeutig auf die hydrokolloidalen Eigenschaften des schleimstoffreichen Extrakts eines nicht-newtonsches Fluids zurück zu führen. Der erfindungsgemäße Extrakt weist durch den erhöhten Schleimstoffgehalt das oben erläuterte nicht-newtonsche Fliessverhalten auf bzw. ist ein nicht-newtonsches Fluid. Die rheologischen Eigenschaften können als Beleg herangezogen werden, und zwar als ein Differenzierungs- und Qualitätsmerkmal für die besondere Eignung als Feuchtigkeitsmittel, da der erfindungsgemäß ausgebildete hydrokolloidale Charakter des Extrakts für sein wasserbindendes Vermögen verantwortlich ist.

Das erfindungsgemäße Verfahren erlaubt die vorteilhafte Anreicherung von Schleimstoffen, welche im Wesentlichen die eigenen nativ in Opuntia ficus indica enthaltenen Polysaccharide umfassen. Es handelt sich hierbei um Extrakteigene Polysaccharide, die aufgrund des erfindungsgemäßen Verfahrens aus dem Pflanzenstoff ausgelöst und als Schleimstoff angereichert werden, jedoch nicht zugesetzt werden müssen.

Daher betrifft die Erfindung ebenfalls einen Opuntia ficus indica-Extrakt, insbesondere einen hydrokolloidalen Extrakt erhältlich aus dem erfindungsgemäßen Verfahren.

In einer weiteren Ausführungsform weist der erfindungsgemäße hydrokolloidale Extrakt eine Viskosität, insbesondere eine Null-Scherviskosität von 5,55 mPa.s und mehr, insbesondere 25,4 mPa.s und mehr auf.

In einer weiteren Ausführungsform weist der erfindungsgemäße hydrokolloidale Extrakt eine Viskosität vs. Schergeschwindigkeit von 4,51 mPa.s bei 10E2 s⁻¹ und mehr, insbesondere 8,1 mPa.s bei 10E2 s⁻¹ und mehr auf.

In einer weiteren Ausführungsform weist der erfindungsgemäße hydrokolloidale Extrakt eine Viskosität vs. Schergeschwindigkeit von 3,19 mPa.s bei 10E3 s⁻¹ und mehr, insbesondere 4,44 mPa.s bei 10E3 s⁻¹ und mehr auf.

Ein erfindungsgemäßes Kosmetikum weist daher keine zusätzlichen Zucker auf, wie z.B. Maltodextrin o.a., sondern verfügt über einen hydrokolloidalen Extrakt mit einzigartigen Schleimstoffen enthaltend insbesondere Polysaccharide mit hervorragenden Eigenschaften zur Aufrechterhaltung der Hautfeuchtigkeit (Moisturizing). Folglich wird erfindungsgemäß ein hervorragendes hautfeuchtigkeitsbindendes und -erhaltendes Mittel erhalten. In allen Altersgruppen ist die Versorgung der Haut mit Feuchtigkeit wesentlich. Daher ist es besonders vorteilhaft, wenn Stoffe und Zubereitungen eine hohe Feuchtigkeitsanreicherung aufweisen. Reduzierte Hautfeuchtigkeit führt zu unerwünschten Hautzuständen, die u.a. den Alterungsprozess beschleunigen können.

Daher betrifft die Erfindung ebenfalls ein Anti-Ageing Mittel aufweisend den erfindungsgemäßen hydrokolloidalen Extrakt.

In einer weiteren Ausführungsform kann die erfindungsgemäße kosmetische oder dermatologische Zubereitung zur topischen Verwendung in Form eines Gels, eines Sprays, einer Creme oder einer Lotion erfolgen, wobei vorzugsweise wässrige Systeme verwendet werden.

Emulgatoren können vorteilhaft gewählt werden aus der Gruppe der nichtionischen, anionischen, kationischen oder amphoteren Emulgatoren.

Erfindungsgemäß bevorzugt sind natürlich vorkommende Emulgatoren, zu denen beispielsweise Bienenwachs, Wollwachs, Lecithin und Sterole u.a. gehören, die ebenfalls bei der Herstellung einer erfindungsgemäßen Zusammensetzung eingesetzt werden können.

Die erfindungsgemäßen kosmetischen Zusammensetzungen können weiterhin kosmetische Hilfsstoffe enthalten, wie sie üblicherweise in solchen Zubereitungen verwendet werden, z. B. Antioxidantien, Radikalfänger, Konservierungsmittel, Bakterizide, Parfüme, Farbstoffe, Pigmente, die eine färbende Wirkung haben, Verdickungsmittel, oberflächenaktive Substanzen, weichmachende, oder andere übliche Bestandteile einer kosmetischen Formulierung wie Alkohole, Polyole, Polymere, Schaumstabilisatoren, Elektrolyte, organische Lösemittel.

In einer weiteren besonderen Ausführungsform besteht die erfindungsgemäße Zusammensetzung vorzugsweise aus natürlich vorkommenden oder naturnahen, respektive naturidentischen Inhaltsstoffen.

In einer weiteren bevorzugten Ausführungsform weist die erfindungsgemäße kosmetische Zusammensetzung zusätzlich ein

Feuchtigkeitsmittel auf, insbesondere ausgewählt aus der Gruppe Hyaluronsäure, Glycerin, Aloe Vera Barbadensis Saft, Betain, Sorbit, Xylit, 1,2-Propylenglykol, Polyalkohole.

In einer weiteren Ausführungsform kann die erfindungsgemäße kosmetische Zusammensetzung auf einen festen Träger aufgebracht sein, wie z.B. Feuchttücher.

Nachfolgende Ausführungsbeispiele und Figuren dienen zur Erläuterung der Erfindung, ohne die Erfindung auf diese Beispiele zu beschränken.

### Beispiel 1

Die feuchtigkeitsspendenden Eigenschaften des Extraktes aus Opuntia ficus indica Kladodien wurden in einem Vergleichsversuch mit kommerziell erhältlichen Extrakten (Benchmarks) in einer in-vivo Studie an 29 Probanden durchgeführt. Für den Vergleich wurden folgende Marktprodukte verwendet: Benchmark 1 - Opuntia Biocomplex SH von Bionap^{®} in Pulverform und Benchmark 2 - flüssige Extrakt Aquapuntia^{®} von Biocosmethic. Beide Benchmarks werden aus dem Saft von frischen Opuntia ficus indica Kladodien hergestellt und es wird lediglich jeweils ein Presssaft erhalten. Die jeweiligen Produkte wurden entsprechend den Empfehlungen an die Einsatzkonzentration des jeweiligen Herstellers in eine kosmetische Modellrezeptur eingearbeitet, die aufgrund ihrer Zusammensetzung keine feuchtigkeitsspendenden Eigenschaften aufweist und zusätzlich als Placebo verwendet wurde.

| **Inhaltsstoff (INCI)** | **Gewichtsanteil** |
|---|---|
| Aqua | **ad 100%** |
| Glyceryl stearate citrate | **7.00%** |
| Caprylic/Capric triglyceride | **6.00%** |
| Octyldodecanol | **4.00%** |
| Cetyl alcohol | **0.86%** |
| Benzyl Alcohol | **0.10%** |
| Benzoic Acid | **0.10%** |
| Sorbic acid | **0.05%** |
| Citric acid | **0.05%** |

Die feuchtigkeitsspendenden Eigenschaften der Testrezepturen wurden mit einem Corneometer (Corneometer CM^{®} 825 (Courage & Khazaka electronic) mit Hilfe der kapazitiven Bestimmung des Wechselstromwiderstandes bestimmt. Die ermittelten Werte korrelieren mit der Hautfeuchte der oberen Hautschichten. Die Signifikanz der Resultate wurde mittels p-Wert evaluiert. Die Modellrezeptur wurde mit 2% des erfindungsgemäßen schleimstoffangereicherten Opuntia ficus indica Extrakts versetzt, und mit der gleichen Konzentration von Benchmark 1 und Benchmark 2 verglichen. Tabelle 1 zeigt nach Applikation innerhalb von 2h, 4h und 24h auf der Vorderarmhaut des Probanden den Anstieg der Feuchtigkeit (Hydration) der Haut(schichten).

**Tabelle 1:**

| | Anstieg der Hydratation | | |
|---|---|---|---|
| | nach 2h | nach 4h | nach 24h |
| Hydrokolloidaler Extrakt mit angereichertem Schleimstoffanteil | 13% | 14% | 8% |
| Benchmark 1 | 5% | 5% | nicht signifikant |
| Benchmark 2 | 6% | 5% | -4% |

Die Resultate zeigen, dass die kosmetischen Modellrezepturen einen signifikanten Anstieg in der Hydratation nach 2h, 4h und 24h aufweisen, insbesondere ist der Anstieg stark erhöht, wenn sie mit einem erfindungsgemäßen schleimstoffangereicherten hydrokolloidalen Extrakt versetzt wird. Die kommerziell verfügbaren Benchmarks zeigen bei gleicher Einsatzkonzentration einen deutlich reduzierten oder einen nicht signifikanten oder sogar einen negativen Effekt.

### Beispiel 2:

Der hydrokolloidale Charakter des erfindungsgemäßen flüssigen Extrakts aus Opuntia ficus indica korreliert mit dem ausreichenden Gehalt an aus den Kladodien extrahierten Schleimstoffen und spiegelt sich sowohl in der Viskosität als auch im Fliessverhalten des Extraktes wider. Diese Eigenschaften wurden in einem Vergleichsversuch mit kommerziell erhältlichen Extrakten (Benchmarks) überprüft. Für den Vergleich wurden folgende Marktprodukte verwendet: Benchmark 1 - Opuntia Biocomplex SH von Bionap^{®} in Pulverform und Benchmark 2 - flüssige Extrakt Aquapuntia^{®} von Biocosmethic. Der Trockenextrakt (Benchmark 1) enthält zusätzlich als Träger Maltodextrin und wird vom Hersteller für den Einsatz in topischen Formulierungen zu 2-5% w/w empfohlen. Für den Vergleich wurden wässrige Lösungen des Benchmarks 1 mit 2%, 3% und 5% des Trockenextrakts frisch zubereitet. Das Benchmark 2 wurde in seiner Lieferform als Flüssigextrakt direkt verwendet. Die Vergleichsversuche wurden mit Hilfe eines Auslaufbechers durchgeführt. Diese Vorrichtung umfasst einen konisch zulaufenden Behälter mit einer Auslauföffnung im Boden samt einem definierten Durchmesser, montiert auf einem 3-Fussgestell. Der Auslaufbecher wird randvoll mit der Testflüssigkeit gefüllt, während die Bodenöffnung manuell verschlossen bleibt. Sobald die Bodenöffnung geöffnet wird, wird die Auslaufzeit der Testflüssigkeit bis zur Entleerung des Bechers gemessen. Experimentell ermittelte Auslaufzeiten korrelieren mit dem rheologischen Charakter der Extrakte, welche in Beispiel 3 beschrieben sind.

**Tabelle 2: Auslaufzeiten der Opuntia ficus indica Extrakte**

| **Extrakt** | Erfindungsgemäss hergestellter Opuntia ficus indica Extrakt | Benchmark 1 | Benchmark 1 | Benchmark 1 | Benchmark 2 |
|---|---|---|---|---|---|
| | | 2% Lösung in Wasser | 3% Lösung in Wasser | 5% Lösung in Wasser | |
| **Auslaufzeit** | min. 20s | 10s | 10s | 10s | 10s |

Die Auslaufzeit der Benchmarks 1 und 2 ist wesentlich kürzer als die Auslaufzeit des erfindungsgemäßen Extrakts. Dies gibt einen Hinweis auf einen deutlich niedrigeren Schleimstoffgehalt der beiden geprüften Benchmarks. Dies ist darin begründet, dass bei einer mechanischen Saftpressung nicht ausschöpfend Schleimstoffe gewonnen werden, und eben kein hydrokolloidaler Extrakt erhalten wird.

Extraktionsversuche an getrockneten Kladodien zeigen zudem, dass die Zugabe von 30 % igem Ethanol zu einem schleimstoffreichen Extrakt zu sichtbaren, flockenartigen Ausfällungen führt. Bei Zugabe von 30 % igem Ethanol zu den Benchmarks 1 und 2 wurde lediglich eine homogene Eintrübung beobachtet, die nach 24h zu einer Sedimentbildung führt. Die Abwesenheit von Flocken schließt ein hydrokolloidales Netzwerk der Benchmarks aus und bestätigt, dass die Benchmarks 1 und 2 allenfalls geringe Mengen bis Spuren an Schleimstoffen aufweisen.

### Beispiel 3:

### Viskositäts- und Schergeschwindigkeitsmessung mit einem Rheometer (DHR2, TA Instruments)

Die Figur 1a zeigt anhand der erfindungsgemäß hergestellten Extrakte, bezeichnet als Plan 16, Plan 22 und Plan 26 im Vergleich zu den kommerziell erhältlichen Benchmark-Extrakten aus Opuntia ficus indica Kladodien (Muster AC-84 (Benchmark 1 (supra)) und AC-92 (Benchmark 2 (supra)), ein Verhalten eines nicht-newtonschen Fluids, während Muster AC-84 und AC-92 ein newtonsches Fluidverhalten zeigen (Messung bei Normalbedingungen).

**Tabelle 3: Viskosität vs. Schergeschwindigkeit**

| **T** | **Viskosität (mPa.s) bei 10E2 s-1** | | | **Viskosität (mPa.s) bei 10E3 s-1** | | |
|---|---|---|---|---|---|---|
| Probe | **Durchlauf 1** | **Durchlauf 2** | **Mittelwert** | **Durchlauf1** | **Durchlauf 2** | **Mittelwert** |
| AC-84 | 2.24 | 2.26 | 2.25 | 2.34 | 2.36 | 2.35 |
| AC-92 | 1.45 | 1.40 | 1.43 | 1.71 | 1.66 | 1.68 |
| Plan 16 | 8.10 | 8.67 | **8.38** | 4.44 | 4.78 | **4.61** |
| Plan 22 | 4.51 | 4.61 | **4.56** | 3.19 | 3.28 | **3.24** |
| Plan 26 | 17.6 | 17.4 | **17.5** | 7.50 | 7.40 | **7.45** |

Figur 1b zeigt mittels einer Oszillationsmesstechnik, dass der Phasenverschiebungswinkel in den schleimstoffreichen hydrokolloidalen Extrakten (Muster: Plan 16, Plan 22 und Plan 26) im breiten Oszillationsbereich reduziert und konstant bei 45°-65° liegt, welches auf die tendenziell elastischen und strukturierten hydrokolloidalen Netzwerkstrukturen hinweist. Die rheologischen Eigenschaften, insbesondere das nicht-newtonsche Fliessverhalten unterscheidet sich deutlich von den kommerziell erhältlichen Opuntia ficus indica - Extrakten (Muster AC-84 (Benchmark 1 (supra)) und AC-92 (Benchmark 2 (supra)). Muster AC-84 und AC-92 zeigen bei den ausgesetzten Oszillationen eine wesentlich niedrigere Null-Scherviskosität (Messung bei Normalbedingungen).

**Tabelle 4:**

| **Null-Scherviskosität (mPa.s)** | | | |
|---|---|---|---|
| Probe | **Durchlauf 1** | **Durchlauf 2** | **Mittelwert** |
| AC-84 | 2.23 | 2.53 | 2.38 |
| AC-92 | 1.52 | 1.74 | 1.63 |
| Plan 16 | 25.4 | 27.5 | 26.4 |
| Plan 22 | 5.97 | 5.55 | 5.76 |
| Plan 26 | 73.4 | 104 | 88.9 |

## Patentansprüche

1. Verfahren zur Herstellung eines hydrokolloidalen Extraktes aus Opuntia ficus indica, **dadurch gekennzeichnet, dass**
a.) aus Pflanzenmaterial von Opuntia ficus indica ein wässriger oder wässrig-alkoholischer Auszug mit maximal 40 % (m/m) Alkohol hergestellt wird,
b.) der erhaltene Auszug in der Wärme bei 60 - 90 Grad Celsius für eine Stunde oder länger behandelt wird,
c.) nach Abkühlung mazeriert und gefiltert wird.

2. Verfahren zur Herstellung eines hydrokolloidalen Extraktes aus Opuntia ficus indica nach Anspruch 1, wobei der Alkohol ein C1-C4 oder C1-C3 Alkohol ist, vorzugsweise Ethanol (C2).

3. Verfahren zur Herstellung eines hydrokolloidalen Extraktes aus Opuntia ficus indica nach Anspruch 1, wobei der Alkohol ein mehrwertiger Alkohol ist, insbesondere ausgewählt aus der Gruppe Glykol, 1,2-Propandiol, Glycerin oder Polyol.

4. Verfahren zur Herstellung eines hydrokolloidalen Extraktes aus Opuntia ficus indica nach Anspruch 1 oder Anspruch 2, wobei der wässrig-alkoholische Auszug mit maximal 30 % (m/m) Alkohol oder 20 % (m/m) Alkohol, insbesondere Ethanol hergestellt wird.

5. Verfahren zur Herstellung eines hydrokolloidalen Extraktes aus Opuntia ficus indica nach einem der vorhergehenden Ansprüche 1 bis 4, **dadurch gekennzeichnet, dass** das Filtern in Schritt c.) mit ein oder mehreren Sieben erfolgt, wobei die Sieböffnungen 60-300 µm betragen.

6. Verfahren zur Herstellung eines hydrokolloidalen Extraktes aus Opuntia ficus indica nach einem der vorhergehenden Ansprüche 1 bis 4, wobei das Pflanzenmaterial von Opuntia ficus indica Kladodien, insbesondere getrocknete Kladodien sind.

7. Hydrokolloidaler Extrakt aus Opuntia ficus indica erhältlich nach einem Verfahren gemäß einem der Ansprüche 1 bis 6.

8. Hydrokolloidaler Extrakt aus Opuntia ficus indica nach Anspruch 7, aufweisend ein nicht-newtonsches Fliessverhalten.

9. Hydrokolloidaler Extrakt aus Opuntia ficus indica nach Anspruch 7 oder Anspruch 8, aufweisend eine Viskosität, insbesondere eine Null-Scherviskosität von 5,55 mPa.s und mehr, insbesondere 25,4 mPa.s und mehr.

10. Hydrokolloidaler Extrakt aus Opuntia ficus indica nach Anspruch 7 oder Anspruch 8, aufweisend eine Viskosität vs. Schergeschwindigkeit von 4,51 mPa.s bei 10E2 s⁻¹ und mehr, insbesondere 8,1 mPa.s bei 10E2 s⁻¹ und mehr oder 3,19 mPa.s bei 10E3 s⁻¹ und mehr, insbesondere 4,44 mPa.s bei 10E3 s⁻¹ und mehr.

11. Kosmetikum enthaltend einen hydrokolloidalen Extrakt nach einem der Ansprüche 7 bis 10.

12. Feuchtigkeitsspendendes Mittel oder Anti-Ageing Mittel enthaltend einen hydrokolloidalen Extrakt nach einem der Ansprüche 7 bis 10.

13. Kosmetikum nach Anspruch 11 oder feuchtigkeitsspendendes Mittel nach Anspruch 12 weiter aufweisend ein Feuchtigkeitsmittel, insbesondere ausgewählt aus der Gruppe Hyaluronsäure, Glycerin, Aloe Vera Barbadensis Saft, Betain, Sorbit, Xylit, 1,2-Propylenglykol, Polyalkohole.

14. Kosmetikum nach Anspruch 11 oder feuchtigkeitsspendendes Mittel nach Anspruch 12 in Form eines Gels, eines Sprays, einer Creme oder einer Lotion.

15. Kosmetikum nach Anspruch 11 oder feuchtigkeitsspendendes Mittel nach Anspruch 12 umfassend einen festen Träger, insbesondere Feuchttücher.

## Claims

1. Process for preparing a hydrocolloidal extract from Opuntia ficus indica, **characterised in that**
a.) an aqueous or aqueous-alcohol extract with a maximum of 40 % (m/m) alcohol is prepared from plant material of Opuntia ficus indica,
b.) the obtained extract is treated under heat at 60 - 90 degrees Celsius for one hour or longer,
c.) is macerated and filtered after cooling.

2. The process for preparing a hydrocolloidal extract of Opuntia ficus indica according to claim 1, wherein the alcohol is a C1-C4 or C1-C3 alcohol, preferably ethanol (C2) .

3. The process for preparing a hydrocolloidal extract of Opuntia ficus indica according to claim 1, wherein the alcohol is a polyvalent alcohol, in particular selected from the group of glycol, 1,2-propanediol, glycerol or polyol.

4. The process for preparing a hydrocolloidal extract of Opuntia ficus indica according to claim 1 or claim 2, wherein the aqueous-alcohol extract is prepared with a maximum of 30% (m/m) alcohol or 20% (m/m) alcohol, in particular ethanol.

5. The process for preparing a hydrocolloidal extract of Opuntia ficus indica according to one of preceding claims 1 to 4, **characterised in that** the filtration in step c.) is carried out with one or more sieves, wherein the sieve openings are 60-300 µm.

6. The process for preparing a hydrocolloidal extract of Opuntia ficus indica according to one of preceding claims 1 to 4, wherein the plant material of Opuntia ficus indica is cladodes, in particular dried cladodes.

7. A hydrocolloidal extract of Opuntia ficus indica obtainable by a process according to one of claims 1 to 6.

8. The hydrocolloidal extract of Opuntia ficus indica according to claim 7, having a non-Newtonian flow behaviour.

9. The hydrocolloidal extract of Opuntia ficus indica according to claim 7 or claim 8, having a viscosity, in particular a zero shear viscosity, of 5.55 mPa.s and more, in particular 25.4 mPa.s and more.

10. The hydrocolloidal extract of Opuntia ficus indica according to claim 7 or claim 8, having a viscosity vs. shear rate of 4.51 mPa.s at 10E2 s⁻¹ and more, in particular 8.1 mPa.s at 10E2 s⁻¹ and more or 3.19 mPa.s at 10E3 s⁻¹ and more, in particular 4.44 mPa.s at 10E3 s⁻¹ and more.

11. A cosmetic containing a hydrocolloidal extract according to one of claims 7 to 10.

12. A moisturising agent or anti-ageing agent containing a hydrocolloidal extract according to one of claims 7 to 10.

13. The cosmetic according to claim 11 or moisturising agent according to claim 12, further comprising a moisturiser, in particular selected from the group of hyaluronic acid, glycerol, aloe vera barbadensis juice, betaine, sorbitol, xylitol, 1,2-propylene glycol, polyalcohols.

14. The cosmetic according to claim 11 or the moisturising agent according to claim 12 in the form of a gel, a spray, a cream or a lotion.

15. The cosmetic according to claim 11 or the moisturising agent according to claim 12 comprising a solid carrier, in particular wet wipes.
The invention relates to a cosmetic composition containing a hydrocolloidal extract of Opuntia ficus indica, wherein mucilages are selectively enriched. Furthermore, the invention relates to a hydrocolloidal extract of Opuntia ficus indica, a process for its preparation, and its use, in particular in cosmetics.

## Revendications

1. Procédé de préparation d'un extrait hydrocolloïdal d'Opuntia ficus indica, **caractérisé en ce que**
a.) on prépare à partir de matériel végétal d'Opuntia ficus indica un extrait aqueux ou aqueux-alcoolique avec au maximum 40 % (m/m) d'alcool,
b.) l'extrait obtenu est traité à la chaleur à 60 - 90 degrés Celsius pendant une heure ou plus,
c.) après refroidissement, on procède à une macération et à une filtration.

2. Procédé de préparation d'un extrait hydrocolloïdal d'Opuntia ficus indica selon la revendication 1, dans lequel l'alcool est un alcool en C1-C4 ou en C1-C3, de préférence l'éthanol (C2).

3. Procédé de préparation d'un extrait hydrocolloïdal d'Opuntia ficus indica selon la revendication 1, dans lequel l'alcool est un polyalcool, en particulier choisi dans le groupe constitué par le glycol, le 1,2-propanediol, le glycérol ou le polyol.

4. Procédé de préparation d'un extrait hydrocolloïdal d'Opuntia ficus indica selon la revendication 1 ou la revendication 2, dans lequel l'extrait hydro-alcoolique est préparé avec au maximum 30 % (m/m) d'alcool ou 20 % (m/m) d'alcool, en particulier de l'éthanol.

5. Procédé de préparation d'un extrait hydrocolloïdal d'Opuntia ficus indica selon l'une quelconque des revendications précédentes 1 à 4, **caractérisé en ce que** la filtration à l'étape c.) est effectuée avec un ou plusieurs tamis, les ouvertures de tamis étant de 60-300 µm.

6. Procédé de préparation d'un extrait hydrocolloïdal d'Opuntia ficus indica selon l'une quelconque des revendications précédentes 1 à 4, dans lequel le matériel végétal d'Opuntia ficus indica est constitué de cladodes, en particulier de cladodes séchés.

7. Extrait hydrocolloïdal d'Opuntia ficus indica pouvant être obtenu par un procédé selon l'une quelconque des revendications 1 à 6.

8. Extrait hydrocolloïdal d'Opuntia ficus indica selon la revendication 7, présentant un comportement d'écoulement non newtonien.

9. Extrait hydrocolloïdal d'Opuntia ficus indica selon la revendication 7 ou la revendication 8, présentant une viscosité, en particulier une viscosité à cisaillement nul, de 5,55 mPa.s et plus, en particulier de 25,4 mPa.s et plus.

10. Extrait hydrocolloïdal d'Opuntia ficus indica selon la revendication 7 ou la revendication 8, présentant une viscosité vs vitesse de cisaillement de 4,51 mPa.s à 10E2 s⁻¹ et plus, en particulier de 8,1 mPa.s à 10E2 s⁻¹ et plus ou de 3,19 mPa.s à 10E3 s⁻¹ et plus, en particulier de 4,44 mPa.s à 10E3 s⁻¹ et plus.

11. Produit cosmétique contenant un extrait hydrocolloïdal selon l'une quelconque des revendications 7 à 10.

12. Composition hydratante ou anti-âge contenant un extrait hydrocolloïdal selon l'une quelconque des revendications 7 à 10.

13. Produit cosmétique selon la revendication 11 ou produit hydratant selon la revendication 12, comprenant en outre un agent hydratant, en particulier choisi dans le groupe constitué par l'acide hyaluronique, la glycérine, le jus d'Aloe Vera Barbadensis, la béta ne, le sorbitol, le xylitol, le 1,2-propylèneglycol, les polyalcools.

14. Produit cosmétique selon la revendication 11 ou produit hydratant selon la revendication 12 sous la forme d'un gel, d'un spray, d'une crème ou d'une lotion.

15. Produit cosmétique selon la revendication 11 ou produit hydratant selon la revendication 12 comprenant un support solide, en particulier des lingettes humides.
